(19) Europäisches Patentamt
European Patent Office
Office européen des brevets

(11) **EP 3 730 042 A1**

(12) **EUROPEAN PATENT APPLICATION**

(43) Date of publication:
**28.10.2020 Bulletin 2020/44**

(51) Int Cl.:
**A61B 5/00** (2006.01)     **G01B 9/02** (2006.01)

(21) Application number: **20155299.9**

(22) Date of filing: **04.02.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.02.2019   US 201962802549 P**

(71) Applicant: **Canon U.S.A., Inc.
Irvine, CA 92618-3731 (US)**

(72) Inventor: **YAMADA, Daisuke
Irvine,, CA California 92618 (US)**

(74) Representative: **TBK
Bavariaring 4-6
80336 München (DE)**

(54) **APPARATUS FOR REDUCING THERMAL NOISE AND AMBIENT LIGHT NOISE IN FLUORESCENCE IMAGING**

(57)     Apparatus, methods and systems relating to fluorescence imaging, and more particularly, to reducing or eliminating light sensitive and insensitive background noise in fluorescence spectroscopy systems, as well as optical coherence tomography (OCT) systems.

FIG. 1

## Description

### CROSS REFERENCE TO RELATED PATENT APPLICATIONS

This application claims priority from U.S. Provisional Patent Application No. 62/802549 filed on February 7, 2019, in the United States Patent and Trademark Office, the disclosure of which is incorporated herein in its entirety by reference.

### FIELD OF THE DISCLOSURE

The present disclosure relates in general to a fluorescence imaging apparatus, methods and systems, and more particularly, to reducing or eliminating thermal noise and ambient light noise in optical coherence tomography (OCT) and fluorescence spectroscopy.

### BACKGROUND OF THE DISCLOSURE

Optical coherence tomography (OCT) provides high-resolution, cross-sectional imaging of tissue microstructure *in situ* and in real-time, while fluorescence imaging, like near-infrared autofluorescence ("NIRAF"), enables visualization of molecular processes. The integration of OCT and fluorescence imaging in a single catheter provides the capability to simultaneously obtain co-localized anatomical and molecular information from a tissue such as the artery wall. For example, in *"Ex. Vivo catheter-based imaging of coronary atherosclerosis using multimodality OCT and NIRAF excited at 633 nm"* (Biomed Opt Express 2015, 6(4): 1363-1375), Wang discloses an OCT-fluorescence imaging system using He:Ne excitation light for fluorescence and swept laser for OCT simultaneously through the optical fiber probe. Usually, in optical imaging, the signal strength can depend on the distance. The fluorescence signal is weaker when the distance from the imaging probe to the sample is farther. The system disclosed by Wang calibrates the fluorescence light intensity detected by an optical fiber using distance between the optical fiber and the tissue, while OCT can measure the distance.

In a catheter/endoscope based fluorescence system, the catheter itself must emit light (catheter background noise) when excitation light couples into an optical probe of the catheter. This light causes thermal and ambient light noise, collectively referred to as background noise, which must be removed to ensure an accurate measurement. As such, existing background noise removal techniques involve taking measurements after connecting a catheter, wherein the catheter is immersed into a PBS solution to acquire NIRAF background data after utilizing the catheter. The data is then averaged and subtracted from the tissue intensity profiles.

However, this technique leads to several shortcomings, severely hindering the accuracy of the OCT measurement. In particular, the shortcoming include a time gap between the background and signal acquisitions, as well as inaccuracies due to ambient room illumination during background acquisition. As the background noise does not match the background noise at the recording time, due to the time gap to acquire background and signal (Cannot take background when acquiring signals), the amount of background is inaccurate.

With regards to the time gap, acquired background noise does not match the initially recorded background noise because there is a time gap to acquire background and signals. The system cannot calculate background noise when acquiring signals since the catheter is located in the body or close to samples, such that the system cannot block the signals from the sample, such as tissues. Background noise is acquired when the system is setup (startup), typically it happens less than a few minutes after the system is turned on. Then, the system is idle until a physician commences use of the system, which could be greater than 30 minutes after setup/startup. As depicted in Graph 1, as the system remains idle after setup/startup, the temperature inside the system increases which in turn effects the noise from the system.

Graph 1.

By way of example, NIRAF signals are simulated when temperature is 20 degrees at start up, and record MMOCT images at 20 degrees and at 50 degrees. NIRAF signals are elevated with offset dark noise level when the internal temperature of the system increases. This temperature increase could lead to incorrect measurements.

With regards to the ambient room light issues, when the background measurement is acquired, the catheter should be located outside the body because tissue NIRAF signals should not be collected. However, as the catheter is outside the tissue, the catheter may detect ambient light from illumination sources within the room (e.g. - natural and/or man-made light). This ambient light leads to background noise, which causes inaccurate measurements in the system.

Accordingly, it is particularly beneficial to devise apparatus, methods and systems for reducing or eliminating background noise in optical coherence tomography (OCT) and fluorescence spectroscopy.

## SUMMARY

Thus, to address such exemplary needs in the industry, the present disclosure teaches apparatus, systems and methods having an optical device comprising: a console having an attachable optical probe, wherein a first light from the light source in the console couples into the optical probe, a second light is collected from the optical probe, wherein the first light and second light are separated with a beam separator, and the second light is propagated to a detector, and wherein the second light has a longer wavelength then the first light.

In additional embodiments of the disclosure provides an optical system comprising: an optical probe; and a background noise reduction structure, wherein a light sensitive background noise and a light insensitive background noise are acquired by the optical probe, wherein light insensitive background noise is acquired near the same time of acquiring a measurement of a sample, and the light sensitive background noise and the light insensitive background noise are reduced from the sample measurement.

In yet additional embodiments, the background noise is acquired at the same time as dark noise is acquired.

In further embodiments, dark noise is acquired during and until standby mode, or during pullback of the catheter.

The subject innovation further teaches updating background noise and subtracting the updated figure during acquisition of measurements of the tissue sample.

These and other objects, features, and advantages of the present disclosure will become apparent upon reading the following detailed description of exemplary embodiments of the present disclosure, when taken in conjunction with the appended drawings, and provided paragraphs.

## BRIEF DESCRIPTION OF THE DRAWINGS

Further objects, features and advantages of the present disclosure will become apparent from the following detailed description when taken in conjunction with the accompanying figures showing illustrative embodiments of the present invention.

Fig. 1 is a schematic diagram of OCT and fluorescence multi-modality system, according to one or more embodiment of the subject apparatus, method or system.

Fig. 2 depicts a free space beam combiner in PIU, according to one or more embodiment of the subject apparatus, method or system.

Fig. 3 provides a cut-away side perspective view of an exemplary catheter, according to one or more embodiments of the subject apparatus, method or system.

Fig. 4 depicts an exemplary workflow chart, according to one or more embodiment of the subject apparatus, method or system.

Fig. 5 provides various signal processes, according to one or more embodiment of the subject apparatus, method or system.

Fig. 6 is a graph depicting the relationship between noise and temperature as it may relate to one or more embodiment of the subject apparatus, method or system.

Fig. 7 provides an exemplary workflow chart, according to one or more embodiment of the subject apparatus, method or system.

Throughout the Figures, the same reference numerals and characters, unless otherwise stated, are used to denote like features, elements, components or portions of the illustrated embodiments. In addition, reference numeral(s) including by the designation " ' " (e.g. 12' or 24') signify secondary elements and/or references of the same nature and/or kind. Moreover, while the subject disclosure will now be described in detail with reference to the Figures, it is done so in connection with the illustrative embodiments. It is intended that changes and modifications can be made to the described embodiments without departing from the true scope and spirit of the subject disclosure as defined by the appended paragraphs.

## DETAILED DESCRIPTION OF THE DISCLOSURE

Fiber optic catheters and endoscopes have been developed to gain access to internal organs for the purpose of medical prognosis, evaluation, and treatment. For example in the cardiology, OCT (optical coherence tomography), white light back-reflection, NIRS (near infrared spectroscopy) and fluorescence technology have been developed to see structural and/or molecular images of vessels with the use of a catheter. The catheter, which comprises a sheath and an optical probe, is navigated into a coronary artery, near the point of interest. In order to acquire cross-sectional images of tubes and cavities such as vessels, esophagus and nasal cavity, the optical probe is rotated with a fiber optic rotary joint (FORJ). In addition, the optical probe may be simultaneously translated longitudinally during the rotation so that helical scanning pattern images are obtained, providing a three-dimensional rendering of the cavity. This translation is most commonly performed by pulling the tip of the probe back towards the proximal end of the cavity, hence earning the common name 'pullback'.

Imaging of coronary arteries by intravascular OCT and fluorescence system is described in a first embodiment of the subject innovation. In particular, the system is able to obtain reliable florescence signals using the subject noise reduction method(s).

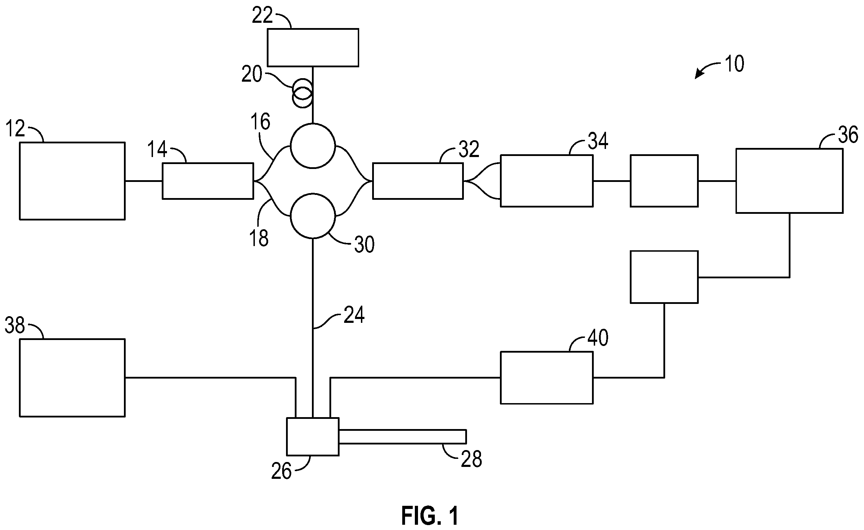

Fig. 1 shows an exemplary OCT and fluorescence multi-modality system 10. Here, an OCT light with a wavelength of around 1.3um, from an OCT light source 12, is delivered and split into a reference arm 16 and a sample arm 18 with a splitter 14. A reference beam 20 is reflected from a reference mirror 22 in the reference arm 16 while a sample beam 24 is reflected and/or scattered from a sample (not shown) through a PIU 26 (patient interface unit) and a catheter 28 in the sample arm 24. Fibers of the PIU 26 and catheter 28 are made of a DCF (double clad fiber). The OCT light 12 illuminates the sample through the core of DCF, and scattered light from the sample are collected and delivered back to the circulator 30 of an OCT interferometer via the PIU 26. The collected light is combined with the reference beam 20 at the combiner 32 and generates interference patterns. The output of the interferometer is detected with the OCT detectors 34 such as photodiodes or multi-array cameras. Then the signals are transferred to a computer 36 to perform signal processing to generate OCT images. The interference patterns are generated only when the path length of the sample arm 18 matches that of the reference arm 16 to within the coherence length of the light source. An excitation light with wavelength of 0.635um, from a fluorescence light source 38, is delivered to the sample through the PIU 26 and the catheter 28. The PIU 26 comprises a free space beam combiner so that the excitation light couples into the common DCF with OCT. The excitation light illuminates the sample from the distal end of the optical probe in the catheter 28. The sample emits auto-fluorescence with broadband wavelengths of 0.65-0.90um, and auto-fluorescence are collected with the catheter 28 and delivered to a fluorescence detector 40 via the PIU 26.

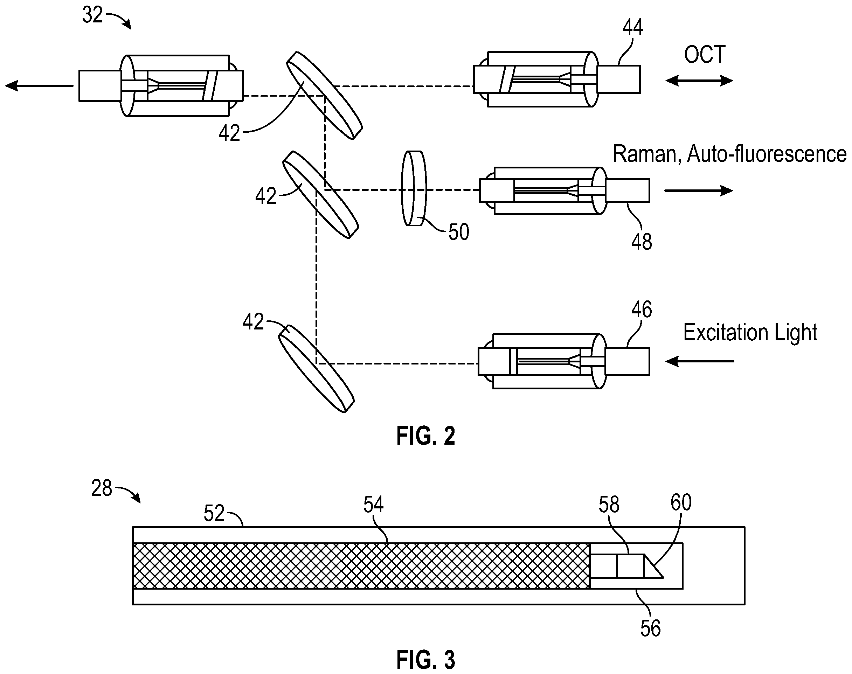

The PIU 26 comprises a free space beam combiner, a FORJ (Fiber Optic Rotary Joint), a rotational motor and a translation motorized stage, and a catheter connector. The FORJ allows uninterrupted transmission of an optical signal while rotating the double clad fiber on the left side along the fiber axis in Figure 2. The FORJ has a free space optical beam coupler to separate a rotor and a stator. The rotator comprises a double clad fiber with a lens to make a collimated beam. The rotor is connected to the optical probe, and the stator is connected to the optical sub-systems. The rotational motor delivers the torque to the rotor. In addition, the translation motorized stage may be used for pullback. A catheter connector is connected to the catheter.

As depicted in Fig. 2 showing a FORJ, the free space beam combiner 32 has dichroic filters 42 to separate different wavelength lights, namely, OCT 44, excitation light 46, and Raman and auto-fluorescence lights (combined) 48. The beam combiner 32 also comprises low-pass filters or band-pass filters 50 in front of the Raman and auto-fluorescence channel to eliminate excitation light because of minimized excitation light noises at the fluorescence detector. The cut-off wavelength of the filter 50 (low-pass or band-pass) is selected from around 645 to 700 nm.

The catheter 28, which comprises a sheath 52, a coil 54, a protector 56 and an optical probe 58, is connected to the PIU 26, as shown in Fig. 3. The optical probe 58 comprises an optical fiber connector, an optical fiber and a distal lens. The optical fiber connector is used to engage with the PIU, and to deliver light to the distal lens. The distal lens is utilized in shaping the optical beam and to illuminate light to the sample, and to collect light from the sample efficiently.

The coil 54 delivers the torque from the proximal end to the distal end by a rotational motor in the PIU 26. There is a mirror 60 at the distal end so that the light beam is deflected outward, at an angle of about 90 degrees to the length of the catheter 28. The coil 54 is fixed with the optical probe so that a distal tip of the optical probe also spins to see omnidirectional views of the inner surface of hollow organs such as vessels. The optical probe 58 comprises a fiber connector at the proximal end, a double clad fiber, and a lens at the distal end. The fiber connector is connected with the PIU 26. The double clad fiber is used to transmit and collect OCT light through the core, and to collect Raman and/or fluorescence from sample through the clad. The lens focuses and collects light to and/or from the sample. The scattered light through the clad is relatively higher than that through the core because the size of the core is much smaller than the clad.

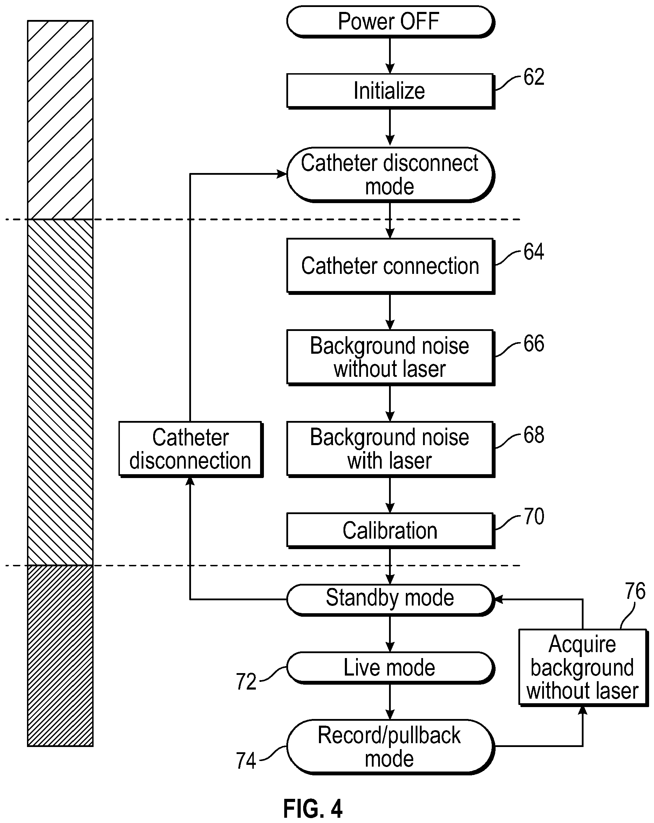

Fig. 4 provides a flow chart detailing measurement workflow, according to one or more embodiment of the subject disclosure. Step 1 in the flowchart involves application software being initialized 62, followed by the system waiting for the catheter connection to be powered on. Step 2 details the system setup process, wherein the user connects the catheter 64 (mechanically and optically), and the system acquires an NIRAF signal (BG1) 66 without the fluorescence light source on, as an external noise such as thermal noise and the noise from ambient light. The NIRAF signal BG1 66 consists of the thermal noise (ThemalN), ambient external light noise (ExtN), and other electrical noise (EleN) such as read-out noise. The thermal noise, the ambient external light noise, the other electrical noise are light insensitive background noise.

$$BG1 = ThermalN_1 + ExtN_1 + EleN \text{ ---- (Equation 1)}$$

In Step 3, the system acquires a second NIRAF signal (BG2) 68 as our device noise with fluorescence light source turned on. The NIRAF signal BG2 68 includes system noise (SysN) excited by the fluorescence light source, thermal noise (ThemalN), external light noise (ExtN), other electrical noise (EleN).

$$BG2 = SysN + ThermalN_1 + ExtN_1 + EleN \text{ ---- (Equation 2)}$$

(Note: The prior art acquire this BG2 before measurements)

The system noise is light sensitive noise.

The system automatically and/or manually calibrates 70 reference arm length to match with the catheter, in Step 4, and the system remains idle until called upon for use by the physician.

Upon implementation of the system by the physician, referred to as Step 5 herein, the user is able to perform live-view image 72 (real-time image) to decide where to acquire MMOCT images. In Step 6, the user initiates a pullback and records 74 OCT signals and NIRAF signals (SG) of the desired lumen. The NIRAF signals (SG) consist of tissue signals (STissue), system noise (SysN) excited by the fluorescence light source, thermal noise (ThemalN), external light noise (ExtN), as well as other electrical noise (EleN).

$$SG = Stissue + SysN + ThermalN_2 + ExtN_2 + EleN \text{ --- (Equation 3)}$$

The thermal noise ($TthN_2$) could be a different value from TthNi because of the time gap. Also, the ambient external light noise ($ExtN_2$) could be different from $ExtN_1$ because the location of catheter may be different (outside of body and inside of body).

Step 7 is where the system acquires NIRAF signal (BG3) 76 without the fluorescence light source.

$$BG3 = ThermalN_2 + ExtN_2 + EleN \text{ ---- (Equation 4)}$$

The noise reduction process involves calculating the calibrated signal (S) in the following equation (Equation 5).

$$Stissue = SG - (BG_2 - BG_1 + BG_3) ---- (Equation 5)$$

With this background noise process, NIRAF measurements become much more accurate and reliable, due to corrections made to account for temperature changes and external light noise.

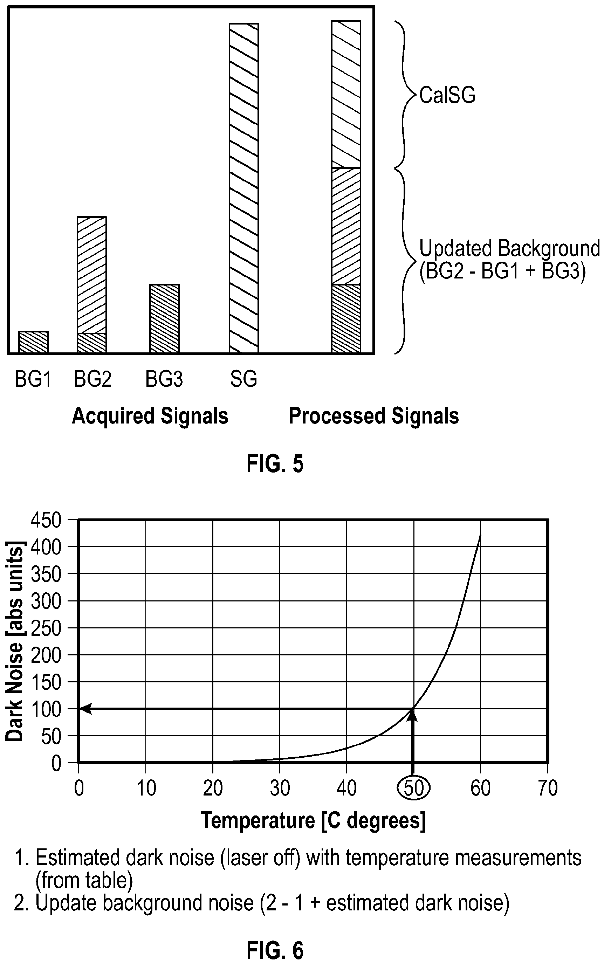

In the second embodiment of the subject disclosure, thermal noise reduction may be pre-determined by calculating and accounting for thermal characteristics. In the first embodiment, the thermal noises are acquired after recording without fluorescence light source 38 turned on (Step 7) with the fluorescence detector 40. Instead of using the fluorescence detector 40, temperature at acquisitions of background and signals can be read. Then, the fluorescence detector 40 may pre-determine the thermal noise depending on the temperature, as shown in Figure 6. Accordingly, the system can estimate the thermal noise and subtract the noise to produce a more accurate measurement.

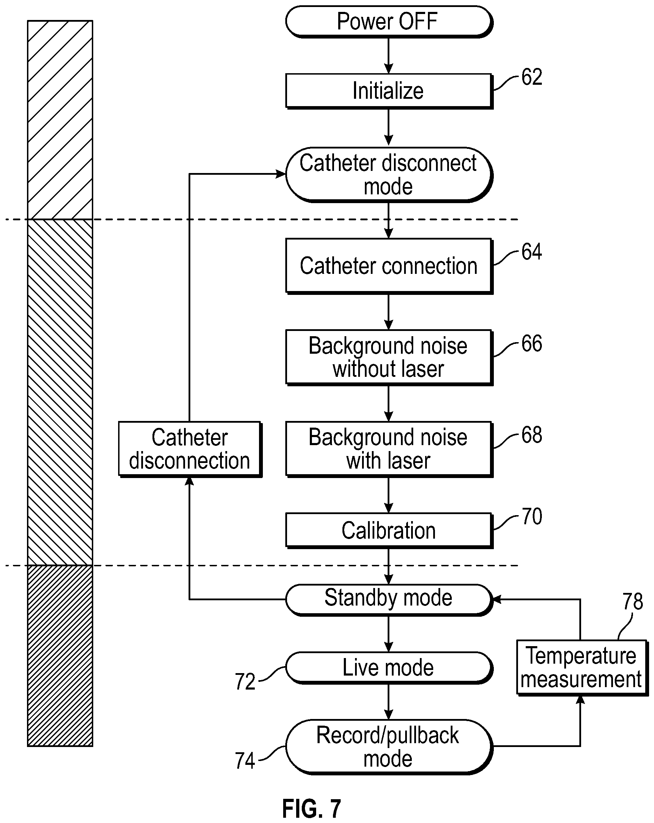

In this method, depicted in Fig. 7, NIRAF background detection is independent of imaging (real-time and/or record imaging) so that the implementation becomes simpler with less constrains. Here, the system has the predetermined function (Equation 6) or the predetermined lookup table to estimate thermal noise (ThermalN) from the temperature (T).

$$ThermalN = Function (T) ---- (Equation 6)$$

As before, Step 1 involves application software initializing 62, wherein the system is waiting for the catheter connection 64 when powered on.

The system setup process includes Step 2, where the user connects the catheter 64 (mechanically and optically), and the system acquires a NIRAF signal (BG1) 66 without the fluorescence light source on. The NIRAF signal BG1 66, consists of the thermal noise (ThemalN) and other electrical noise (EleN) such as read-out noise.

$$BG_1 = ThermalN_1 + EleN ---- (Equation 7)$$

When the electrical noise is small, the thermal noise is able to derived from equation 6 to measure the temperature. In Step 3, the system further acquire NIRAF signal (BG2) 68 as our device noise with fluorescence light source is turned on. The NIRAF signal BG2 68 includes system noise (SysN) excited by the fluorescence light source, the thermal noise (ThemalN) and other electrical noise (EleN).

$$BG_2 = SysN + ThermalN_1 + EleN ---- (Equation 8)$$

Step 4 involves the system automatically and/or manually calibrating 70 the reference arm length to match with the catheter. At this point, the system remains idle until the user calls upon the system to perform measurements of the tissue.

Step 5 initiates measurement of tissue, wherein the user is able to perform live-view image 72 (real-time image) to decide where to acquire MMOCT images. In Step 6, the user perform pullback and records 74 OCT signals and NIRAF signals (SG). The NIRAF signals (SG) consist of tissue signals (Stissue), system noise (SysN) excited by the fluorescence light source, the thermal noise (ThemalN) and other electrical noise (EleN).

$$SG = Stissue + SysN + ThermalN_2 + EleN ---- (Equation 9)$$

The thermal noise (TthN2) could be different value from $TthN_1$ because of the time gap. Step 7 involves the system measuring the temperature ($T_2$) 78, and estimating the thermal noise from pre-determined function (equation 6).

$$BG_3 = ThermalN_2 = Function (T_2) ---- (Equation 10)$$

The system is now ready for the noise reduction process, wherein the calibrated signal (S) is calculated with the following equation.

$$Stissue = SG - (BG2 - BG1 + BG3) ---- (Equation\ 11)$$

With this background noise reduction method, NIRAF measurements become reliable (insensitive to temperature changes), which greatly improves the accuracy and complexity associated with measurements.

In a third embodiment, the NIRAF signal (BG3) is acquired after record/pullback mode. However, it is also possible to acquire before and during recording. Before the measurement, it could be during the live mode or the beginning of record/pullback mode. If this method applies before the live mode, the also thermal/external light noise compensated real-time image at live mode is available. During recording, the system is able to measure the temperature at the same time without any influences.

Apparatus, methods and systems relating to fluorescence imaging, and more particularly, to reducing or eliminating light sensitive and insensitive background noise in fluorescence spectroscopy systems, as well as optical coherence tomography (OCT) systems.

**Claims**

1. An optical system comprising:

    an optical probe for measuring a sample; and
    a background noise reduction structure,

    wherein a light sensitive background noise and a light insensitive background noise are acquired by the optical system,
    wherein light insensitive background noise is acquired near the same time as acquiring the sample measurement by the optical probe, and
    the light sensitive background noise and the light insensitive background noise are reduced from the sample measurement.

2. The optical system of Claim 1, wherein the light sensitive background noise is acquired before acquiring the sample measurement.

3. The optical system of Claim 1, wherein the light sensitive background noise is acquired after acquiring the sample measurement.

4. The optical system of Claim 1, wherein the light sensitive background noise is acquired by blocking reflected light from the sample measurement.

5. The optical system of Claim 1, wherein the light insensitive background noise is acquired without an excitation light.

6. The optical system of Claim 1, further comprising optical coherence tomography for distance calibration.

7. The optical system of Claim 1, wherein light insensitive background noise is calculated from temperature measurement results.

8. The optical system of Claim 1, wherein the near time for acquiring the light insensitive background noise from the measurement of a sample is less than 30 seconds.

9. An optical device comprising:

    a console having an attachable optical probe,
    wherein a first light from the light source in the console couples into the optical probe, a second light is collected from the optical probe,
    wherein the first light and second light are separated with a beam separator, and the second light is propagated to a detector, and
    wherein the second light has a longer wavelength then the first light.

**10.** An optical system comprising:

an optical probe for measuring a sample; and
a background noise reduction structure,

wherein a light sensitive background noise and a light insensitive background noise are acquired by the optical system,
wherein light sensitive background noise is acquired near the same time as acquiring the sample measurement by the optical probe, and
the light sensitive background noise and the light insensitive background noise are reduced from the sample measurement,
wherein the light insensitive background noise is calculated based on the temperature of the system.

**11.** The optical system of Claim 10, wherein the light sensitive background noise is acquired before acquiring the sample measurement.

**12.** The optical system of Claim 10, wherein the light sensitive background noise is acquired after acquiring the sample measurement.

**13.** The optical system of Claim 10, wherein the light sensitive background noise is acquired by blocking reflected light from the sample measurement.

**14.** The optical system of Claim 10, wherein the light insensitive background noise is acquired without an excitation light.

**15.** The optical system of Claim 10, further comprising optical coherence tomography for distance calibration.

**16.** The optical system of Claim 10, wherein the near time for acquiring the light insensitive background noise from the measurement of a sample is less than 30 seconds.

**FIG. 1**

**FIG. 2**

**FIG. 3**

EP 3 730 042 A1

FIG. 4

**Acquired Signals**      **Processed Signals**

CalSG

Updated Background
(BG2 - BG1 + BG3)

BG1    BG2    BG3     SG

**FIG. 5**

1. Estimated dark noise (laser off) with temperature measurements (from table)
2. Update background noise (2 - 1 + estimated dark noise)

**FIG. 6**

```
                    ┌──────────────────────┐
                    │      Power OFF       │
                    └──────────┬───────────┘
                               ↓
                    ┌──────────────────────┐
                    │      Initialize      │──── 62
                    └──────────┬───────────┘
                               ↓
                    ┌──────────────────────┐
              ┌────→│  Catheter disconnect │
              │     │        mode          │
              │     └──────────┬───────────┘
              │                ↓
              │     ┌──────────────────────┐
              │     │  Catheter connection │──── 64
              │     └──────────┬───────────┘
              │                ↓
              │     ┌──────────────────────┐
              │     │   Background noise    │──── 66
              │     │     without laser     │
              │     └──────────┬───────────┘
              │                ↓
  ┌────────────────┐ ┌──────────────────────┐
  │    Catheter    │ │   Background noise    │──── 68
  │  disconnection │ │      with laser       │
  └───────┬────────┘ └──────────┬───────────┘
          ↑                     ↓
          │          ┌──────────────────────┐
          │          │     Calibration      │──── 70
          │          └──────────┬───────────┘
          │                     ↓
          │          ┌──────────────────────┐      ┌────────────────┐
          └──────────│    Standby mode      │←─────│                │──── 78
                     └──────────┬───────────┘      │                │
                                ↓                  │  Temperature   │
                     ┌──────────────────────┐      │  measurement   │
              72 ────│     Live mode        │      │                │
                     └──────────┬───────────┘      └───────┬────────┘
                                ↓                          ↑
                     ┌──────────────────────┐              │
              74 ────│   Record/pullback    │──────────────┘
                     │        mode          │
                     └──────────────────────┘
```

## FIG. 7

Europäisches
Patentamt

European
Patent Office

Office européen
des brevets

**PARTIAL EUROPEAN SEARCH REPORT**

under Rule 62a and/or 63 of the European Patent Convention.
This report shall be considered, for the purposes of
subsequent proceedings, as the European search report

Application Number

EP 20 15 5299

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 174 291 B1 (MCMAHON BRIAN T [US] ET AL) 16 January 2001 (2001-01-16) | 1-3,5,8 | INV. A61B5/00 G01B9/02 |
| Y | * equation 2; column 17, line 36 - column 19, line 10; figure 1A * | 6,7 | |
| X | US 2017/209049 A1 (WANG HAO [US] ET AL) 27 July 2017 (2017-07-27) | 1-6,8 | |
| Y | * paragraphs [0002], [0012], [0056], [0057] * | 7 | |
| Y | UGHI GIOVANNI J ET AL: "Clinical Characterization of Coronary Atherosclerosis With Dual-Modality OCT and Near-Infrared Autofluorescence Imaging", JACC: CARDIOVASCULAR IMAGING, ELSEVIER, AMSTERDAM, NL, vol. 9, no. 11, 9 March 2016 (2016-03-09), pages 1304-1314, XP029800862, ISSN: 1936-878X, DOI: 10.1016/J.JCMG.2015.11.020 * page 1306 * | 6 | |

-/--

TECHNICAL FIELDS
SEARCHED     (IPC)

A61B
G01B

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 17 September 2020 | Biedermann, Benjamin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches
Patentamt

European
Patent Office

Office européen
des brevets

**PARTIAL EUROPEAN SEARCH REPORT**

Application Number

EP 20 15 5299

| | DOCUMENTS CONSIDERED TO BE RELEVANT | | | CLASSIFICATION OF THE APPLICATION (IPC) |
|---|---|---|---|---|
| Category | Citation of document with indication, where appropriate, of relevant passages | | Relevant to claim | |
| Y | JP 2002 330352 A (FUJI PHOTO FILM CO LTD) 15 November 2002 (2002-11-15) * paragraph [0013] * ----- | | 7 | |
| A | EP 2 980 562 A1 (HAMAMATSU PHOTONICS KK [JP]) 3 February 2016 (2016-02-03) * paragraphs [0021] - [0033]; figure 2 * ----- | | 8 | |

| TECHNICAL FIELDS SEARCHED (IPC) |
|---|
| |

EPO FORM 1503 03.82 (P04C10)

Europäisches
Patentamt

European
Patent Office

Office européen
des brevets

**INCOMPLETE SEARCH**
**SHEET C**

Application Number

EP 20 15 5299

```
Claim(s) completely searchable:
      1-8

Claim(s) not searched:
      9-16

Reason for the limitation of the search:

After an invitation pursuant to Rule 62a(1), the applicant indicated
claims 1-8 to be searched.
```

**EP 3 730 042 A1**

## ANNEX TO THE EUROPEAN SEARCH REPORT
## ON EUROPEAN PATENT APPLICATION NO.

EP 20 15 5299

17-09-2020

| Patent document cited in search report | | | Publication date | Patent family member(s) | | | Publication date |
|---|---|---|---|---|---|---|---|
| US 6174291 | B1 | | 16-01-2001 | AT | 412363 | T | 15-11-2008 |
| | | | | AT | 474498 | T | 15-08-2010 |
| | | | | CA | 2322768 | A1 | 16-09-1999 |
| | | | | EP | 1065970 | A1 | 10-01-2001 |
| | | | | EP | 2018824 | A1 | 28-01-2009 |
| | | | | JP | 4477230 | B2 | 09-06-2010 |
| | | | | JP | 2002505900 | A | 26-02-2002 |
| | | | | JP | 2010088929 | A | 22-04-2010 |
| | | | | US | 6174291 | B1 | 16-01-2001 |
| | | | | WO | 9945838 | A1 | 16-09-1999 |
| US 2017209049 | A1 | | 27-07-2017 | EP | 3171766 | A1 | 31-05-2017 |
| | | | | JP | 2017525435 | A | 07-09-2017 |
| | | | | JP | 2020127735 | A | 27-08-2020 |
| | | | | KR | 20170038024 | A | 05-04-2017 |
| | | | | US | 2017209049 | A1 | 27-07-2017 |
| | | | | WO | 2016015052 | A1 | 28-01-2016 |
| JP 2002330352 | A | | 15-11-2002 | NONE | | | |
| EP 2980562 | A1 | | 03-02-2016 | CN | 105074433 | A | 18-11-2015 |
| | | | | EP | 2980562 | A1 | 03-02-2016 |
| | | | | JP | 6198426 | B2 | 20-09-2017 |
| | | | | JP | 2014196953 | A | 16-10-2014 |
| | | | | US | 2016041098 | A1 | 11-02-2016 |
| | | | | WO | 2014155869 | A1 | 02-10-2014 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62802549

**Non-patent literature cited in the description**

- **WANG.** Ex. Vivo catheter-based imaging of coronary atherosclerosis using multimodality OCT and NIRAF excited at 633 nm. *Biomed Opt Express,* 2015, vol. 6 (4), 1363-1375